# NEUE EUROPÄISCHE PATENTSCHRIFT

(11) **EP 1 568 935 B2**
(45) Veröffentlichungstag und Bekanntmachung des Hinweises auf die Entscheidung über den Einspruch: **31.07.2013**
(45) Hinweis auf die Patenterteilung: 21.03.2007
(21) Anmeldenummer: 04004601.3
(22) Anmeldetag: 28.02.2004
(51) Int. Cl.: F21S 8/00, F21S 2/00

(54) **Operationsleuchte**
Surgical operation lamp
Lampe chirurgicale

(43) Veröffentlichungstag der Anmeldung: 31.08.2005
(73) Patentinhaber: TRUMPF Medizin Systeme GmbH + Co. KG, 82178 Puchheim (DE)
(72) Erfinder: Marka, Rudolf, Dr., 81479 München (DE)
(74) Vertreter: Feldmeier, Jürgen

(56) Entgegenhaltungen:
- DE-A- 10 034 594
- DE-U- 20 116 750
- DE-U- 20 214 879
- FR-A- 947 482
- US-A- 6 120 164
- US-A1- 2003 146 719
- US-A1- 2003 185 009
- US-B1- 6 170 963

## Beschreibung

Die Erfindung betrifft eine Operationsleuchte.

Beispielsweise durch die DE 197 29 758 A1 ist eine Operationsleuchte bekannt geworden. Dokument FR 947482 Offenbart eine Operationsleuchte die Sechseckige Lichtmodule mit jeweils einem Leuchtmittel aufweist, die zu einer Facettenstruktur zusammengesetzt sind.

Dokument US 20030146719 offenbart eine Operationsleuchte die aus einzelne Lichtmodulen zusammengesetzt ist.

Großspiegelleuchten der eingangs genannten Art haben den Nachteil, dass die Hindernisse im Lichtfeld abgebildet werden und die Lichtfeldform beeinträchtigen. Die Lichtaustrittsfläche wird unterbrochen.

Einzelne kleinere Lichtaustrittsflächen führen bei aufgelösten Lichtsystemen (z.B. DE 84 05 380 U1) beim Verdecken durch den Operateur zu verstärkten Verlusten in der Beleuchtungsstärke.

Die applikationsbezogene Veränderung der Leuchtfeldform (die auf dem Operationstisch beleuchtete Fläche) ist bei einer Operationsleuchte nach der Lehre des Standes der Technik nicht vorgesehen.

Der Anmelder hat sich die Aufgabe gestellt, eine Operationsleuchte mit einem gleichmäßigen, an unterschiedliche chirurgische Eingriffe anpassbaren Leuchtfeld zu schaffen.

Diese Aufgabe wird durch eine Operationsleuchte gemäß Patentanspruch 1 gelöst. Die zusammensteckbaren Module erlauben eine Anpassung an die Erfordernisse des Operateurs, welcher die Anpassung selbst vornehmen kann. Je nach Kombination der Lichtmodule verändert sich das Leuchtfeld.

Vorteilhafte Weiterbildungen der Erfindung sind in den weiteren Patentansprüchen enthalten:
- Die Ausbildung von Schwenkachsen mithilfe von Gelenken führt zu weiteren individuell durchführbaren Anpassungen der Anordnung der Module, um das Leuchtfeld zu verändern.
- Die Dimmung kann durch mechanische und/oder optische Mittel erreicht werden. Blenden, Linsen oder optische Filter, welche in den Strahlengang hinein bewegt werden können, bewirken eine Veränderung des Lichtstromes. Alternativ kann eine Dimmung durch eine Änderung des der LED zugeführten elektrischen Stroms und/oder der elektrischen Spannung erzeugt werden.

Bevorzugte Ausführungsbeispiele der Erfindung sind in der Zeichnung schematisch dargestellt und wird nachfolgend mit Bezug zu den Figuren der Zeichnung näher erläutert. Es zeigt:
- **Fig. 1**: eine Operationsleuchte;
- **Fig. 2**: mehrere Lichtmodule der Operationsleuchte gemäß Fig. 1;
- **Fig. 3a bis 3d**: Kombinationsmöglichkeiten der Lichtmodule gemäß Fig. 2;
- **Fig. 4a, 4b**: Schwenkmöglichkeiten zweier verbundener Lichtmodule gemäß Fig. 2;
- **Fig. 5**: den näheren Aufbau eines Lichtmoduls gemäß Fig. 2;
- **Fig. 6**: eine weitere Kombinationsmöglichkeit der Module;
- **Fig. 7**: eine weitere Kombinationsmöglichkeit der Module.

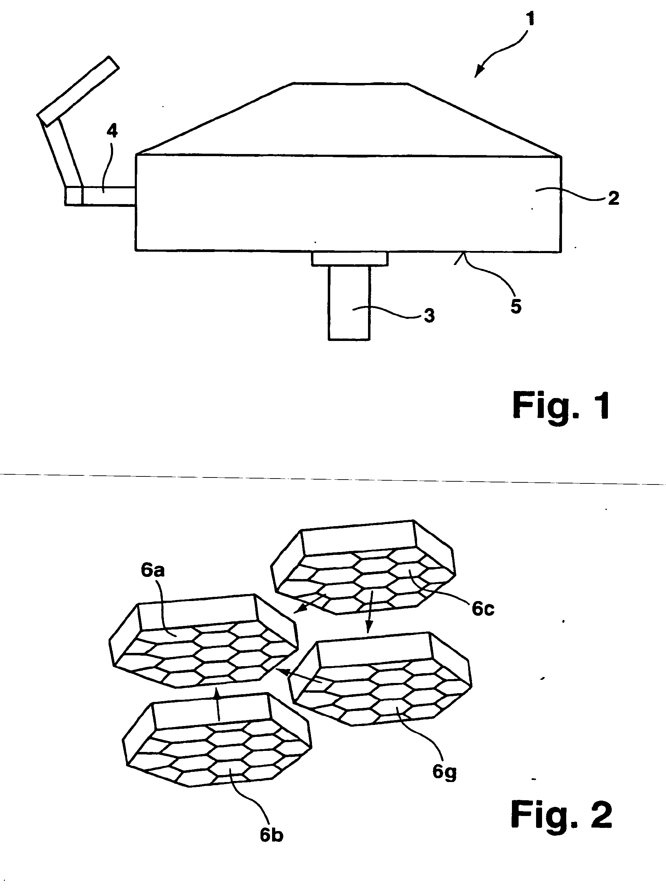

Aus der Seitenansicht einer Operationsleuchte **1** (**Fig. 1**) ist deren prinzipieller an sich bekannter Aufbau ersichtlich. Die Operationsleuchte 1 umfasst einen Leuchtenkörper **2,** welcher in seinem Innenraum in der Fig. 1 nicht sichtbare Leuchtmittel aufweist. Der Leuchtenkörper 2 ist über einen in der Fig. 1 nicht vollständig gezeigten Schwenkarm schwenkbar an einer stationären Halterung an einer Decke oder einer Wand eines Gebäudes oder einer mobilen Einheit befestigt. Der Schwenkarm ist aus mehreren über Gelenke miteinander verbundenen Elementen aufgebaut. Ein mit der Operationsleuchte 1 fest verbundenes Element **4** des Schwenkarms ist in der Fig. 1 lediglich angedeutet. Daher lässt sich die Operationsleuchte 1 in X-, Y-, Z-Richtung dreidimensional bewegen und schwenken. Ein an dem Leuchtenkörper 2 angebrachter Handgriff **3** ermöglicht die Positionierung der Operatiönsleuchte an beliebiger Stelle über einem Operationstisch. Der Handgriff 3 ist lösbar an der Unterseite **5** der Operationsleuchte angebracht.

An der Unterseite 5 tritt Licht aus, um die Operationsstelle auszuleuchten.

**Fig. 2** zeigt, dass als Lichtquelle einzelne Lichtmodule **6a** bis **6g** zusammengesetzt werden können, wie durch die Pfeile angedeutet ist. Die einzelnen Lichtmodule 6a bis 6g umfassen ein Gehäuse, welches an seinen Seitenflächen sowohl mechanische als auch elektronische wie elektrische Verbindungselemente oder Konnektoren aufweist, um Leuchtmittel in den Lichtmodulen anzusteuern und an eine elektrische Stromversorgung anzuschließen. Die Ankopplung der Lichtmodule 6a bis 6g ist über jede Seitenfläche möglich, so dass beliebige Kombinationsmöglichkeiten denkbar sind. Es kann eine Leuchte erzeugt werden, deren Leuchtfeld auf die durchzuführende Operation abgestimmt ist.

Alternativ können die Module mit rückseitigen Konnektoren an einer Trägerplatte befestigt werden.

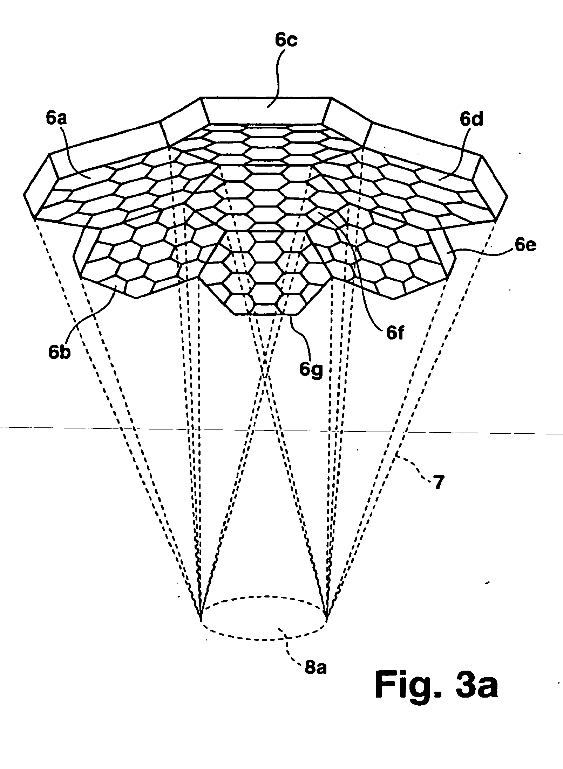

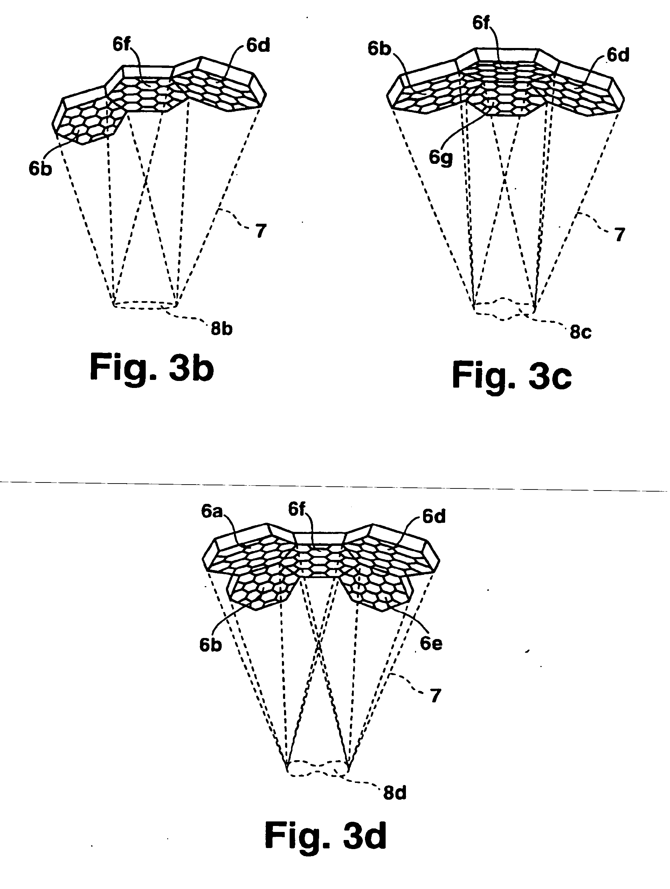

Die **Figuren 3a** bis **3d** zeigen Beispiele für diese Abstimmung der Lichtmodule 6a bis 6g, um durch die Lichtstrahlen **7** Lichtfelder **8a** bis **8d** abzustimmen. Je nach Kombination der Lichtmodule 6a bis 6g wird das Lichtfeld 8a bis 8d eine größere Erstreckung in Längsrichtung oder in der Breite aufweisen. Das Lichtfeld 8a bis 8d kann auch an seinen Enden oder in seiner Mitte unterschiedlich gestaltet werden. Dies bedeutet, dass das Lichtfeld 8a bis 8d die Kontur der Lichtmodulkombination widerspiegelt. Jedes Lichtmodul 6a bis 6g kann selbst eine komplette Operationsstelle ausleuchten. Das Lichtfeld 8a bis 8d ist die auf dem Operationstisch beleuchtete Fläche. Die Lichtmodule 6a bis 6g können durch beliebige Kombination mit weiteren Lichtmodulen zu unterschiedlichsten Gesamtmodulen als Lichtquelle zusammengesetzt werden. Dies verändert die Lichtfeldgröße, die Beleuchtungsstärke und die Lichtfeldform des Lichtfeldes 8a bis 8d.

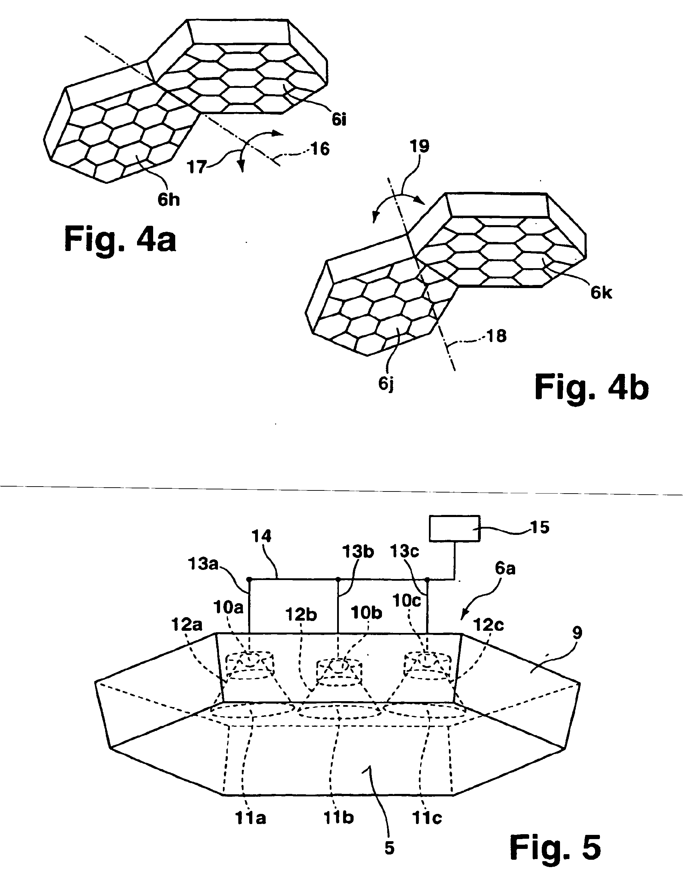

Gemäß **Figuren 4a** bis **4b** sind die Lichtmodule **6h** bis **6k** nicht starr miteinander verbunden, sondern sind mithilfe gelenkiger Verbindungen schwenkbar aneinander gekoppelt. Diese Verbindungen erlauben eine Schwenkbarkeit um die Schwenkachsen **16** und **18** in Doppelpfeilrichtungen **17** und **19.** Fig. 4a zeigt, dass die Seitenflächen, an denen das Licht austritt, aufeinander zu geklappt werden können. Fig. 4b zeigt, dass die Randflächen aufeinander zu geklappt werden können. Neben den gezeigten scharnierartigen Gelenken an einer jeweiligen Verbindungslinie der Lichtmodule 6h bis 6k sind auch Kugelgelenke in einer oder mehreren der sechs Ecken der Lichtmodule 6h bis 6k möglich. Die Gelenke führen dazu, dass bereits kombinierte Lichtmodule 6h bis 6k noch in ihrer Positionierung zueinander verändert werden können. Der Operateur kann die Lichtfelder zusätzlich variieren.

Die Lichtmodule 6a bis 6k sind nahezu randlos aneinander fügbar. Randlos bedeutet im Sinne der Erfindung, dass die Übergänge zwischen den einzelnen Lichtmodulen 6a bis 6k keinen wesentlichen Einfluss auf die optischen Eigenschaften, insbesondere auf den Lichtaustritt in Richtung der Operationsstelle, besitzen. Das erzeugte Licht wird trotz der aus mehreren Lichtmodulen 6a bis 6k zusammengesetzten Lichtquelle als einheitlich wahrgenommen. Jedes Lichtmodul 6a bis 6k umfasst wiederum eine Vielzahl einzelner LEDs, beispielsweise 30 bis 50. Hieraus ergeben sich lichttechnische Vorteile analog zu Großspiegelleuchten wie eine theoretisch optimale Schattenfreiheit durch großflächigen Lichtaustritt.

Die einzelnen Lichtmodule, beispielsweise das in der **Fig. 5** gezeigte Lichtmodul 6a, bestehen alle jeweils aus einem Gehäuse **9** mit mechanischen und/oder elektrischen oder elektronischen Verbindungselementen oder Konnektoren zu benachbarten Lichtmodulen 6b bis 6g. Die Form der Lichtmodule 6a bis 6g ist so gestaltet, dass sie auf einer Kugelfläche mit typischem Radius 1000 mm ohne Zwischenräume angeordnet werden können. Um dies zu erreichen, sind die Lichtmodule 6a bis 6g als Sechsecke ausgebildet. In zusammengesetzter Form ergibt sich eine Art Wabenstruktur oder Facettenstruktur. Die der Operationsstelle zugewandte Fläche der Lichtmodule 6a bis 6g muss auch nicht zwingend eben ausgebildet sein, sondern kann leicht konkav sein, um die Krümmung der Kugelfläche besser nachzubilden. Die optische Achse jedes Lichtmoduls 6a bis 6g weist in den Brennpunkt der Kugelfläche.

Unterschiedliche Lichtfeldformen können durch die Aneinanderreihung von Modulen mit verändertem Anstellwinkel erzeugt werden. Dazu können auch Zwischenelemente zum Einsatz kommen. In jedem Lichtmodul 6a bis 6g sind mehrere, ca. 30 bis 50 LEDs, gleichmäßig verteilt angeordnet, von denen in der Fig. 5 lediglich drei gezeigt und mit Bezugszeichen **10a** bis **10c** bezeichnet sind. Zur Schaffung des flächigen Lichtaustrittes wird der Strahlengang der nahezu punktförmig strahlenden LEDs 10a bis 10c (gestrichelt angedeutet sind beispielhaft die LED-Lichtstrahlen **12a** bis **12c**) durch Linsenelemente **11a** bis **11c** bis zur Lichtaustrittsfläche aufgeweitet und von dort in das Leuchtfeld gelenkt. Dazu ist jeder LED 10a bis 10c ein optisches Linsenelement 11a bis 11c zugeordnet. Die Form der Linsenelemente 11a bis 11c ist so gestaltet, dass sie das Lichtmodul 6a bis zum Rand ausfüllen. Die Linsenelemente 11a bis 11c können zudem über eine Streustruktur zur Vergleichmäßigung des Lichtfeldes verfügen. Die Unterseite 5 der Lichtmodule kann durch eine transparente Scheibe abgedeckt sein.

Die einzelnen Lichtmodule 6a bis 6g bilden zusammen eine Lichtquelle mit einer Farbtemperatur von ca. 4.500 K sowie eines Farbwiedergabeindexes Ra > 93, um eine natürliche Farbdarstellung, beispielsweise des zu operierenden Gewebes, zu erreichen. Deshalb kommen nicht nur LEDs zum Einsatz, welche weißes Licht erzeugen, sondern weitere LEDs, welche farbiges Licht erzeugen. Durch Zumischung farbiger Lichtanteile wie Cyan und Blau wird ein Einbruch im Spektrum wie bei rein weißen LEDs verhindert. Bei konstanter Helligkeit der weißen LEDs können durch ausschließlich stufenlose Dimmung der Intensität der farbigen LEDs die Farbtemperatur und Farbwiedergabe des durch das Gesamtmodul, bestehend aus allen einzelnen Lichtmodulen 6a bis 6g (Gesamtlichtquelle), erzeugten Mischlichts variabel eingestellt werden. Die Lichtstromintensität der farbigen LEDs kann stufenlos verändert werden. Die LEDs 10a bis 10c sind über Stromleitungen **13a** bis **13c** und **14** mit einer Steuereinrichtung **15** verbunden, welche eine elektrische Dimmung des Lichtstroms der LEDs ermöglicht. Die elektrische Dimmung der farbigen LEDs bewirkt eine Änderung der Farbtemperatur und/oder der Farbwiedergabe, was für die Ausleuchtung der Operationsstelle vorteilhaft ist. Hierbei ist denkbar, dass der Operateur die Einstellbarkeit derart nutzt, dass er nach seinen Bedürfnissen farbiges und weißes Licht mischt, um spezielle Gewebearten oder Gewebeveränderungen lichttechnisch hervorzuheben. Der Operateur kann einzelne Gewebearten oder Gewebeveränderungen besser erkennen.

Es wird eine Grundeinstellung einer Farbtemperatur von 4.500 K vorgegeben, welche beim Einschalten der Operationsleuchte automatisch erzeugt wird. Andere je Anwendung der Operationsleuchte gewünschte Farbtemperatur kann mithilfe eines Bedienfeldes oder einer Tastatur einer Steuereinrichtung vom Operateur eingestellt werden. Die notwendigen Einstellparameter können in einem Speicher der Steuereinrichtung abgelegt werden. Weiterhin denkbar ist es, dass der Operateur weitere selbst gewählte Einstellungen zusätzlich abspeichern und diese Einstellungen auch nachträglich verändern kann.

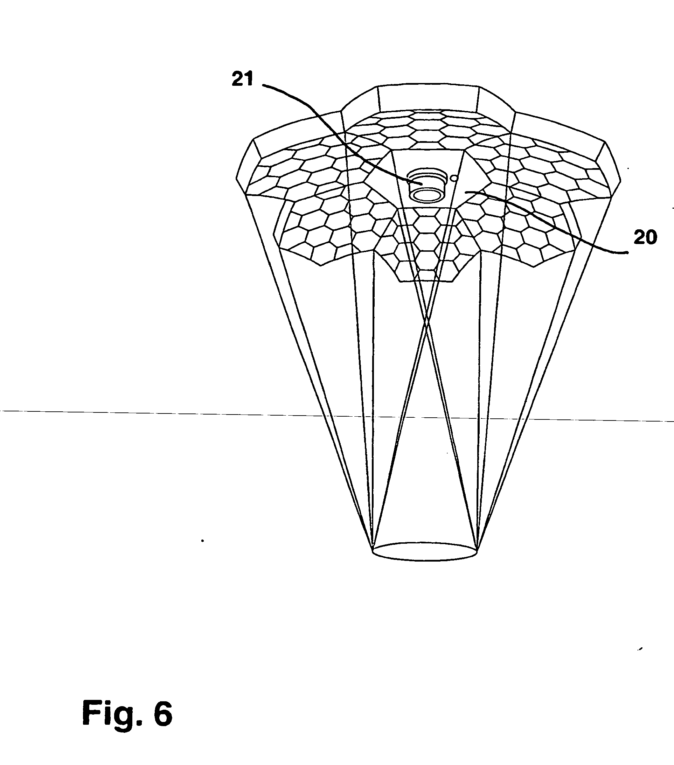

Es ist auch denkbar, weitere Einbau- oder Kombinationsmöglichkeiten wie z.B. ein Kameramodul **20** gemäß **Fig. 6** vorzusehen. Fig. 6 zeigt, dass eines der Module 20, nämlich das zentrale Modul, eine Videokamera **21** trägt. Durch das Einstecken des Moduls 20 werden automatisch die erforderlichen elektrischen und zur Bildverarbeitung geeigneten Verbindungen hergestellt.

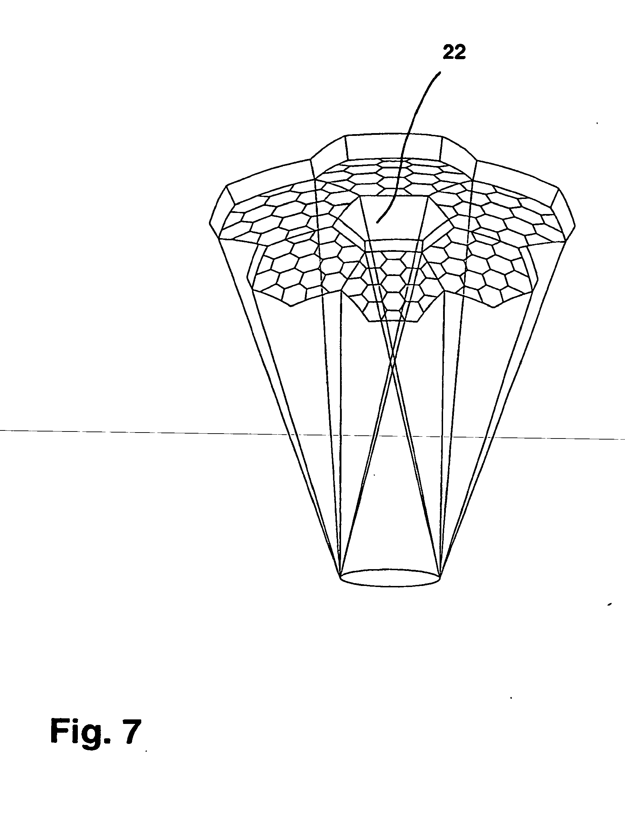

Gemäß **Fig. 7** kann auch eine Leerstelle **22** z.B. in der Mitte der Operationsleuchte vorgesehen sein, um die laminaren Strömung (Laminar Flow) einer eventuell vorhandenen Lüftungsdecke im Operationssaal weniger zu beeinflussen. Die Leerstelle 22 entsteht durch den Nichteinbau eines zentralen Moduls.

### BEZUGSZEICHENLISTE

1 Operationsleuchte
2 Leuchtenkörper
3 Handgriff
4 Element
5 Unterseite
6a Lichtmodul
6b Lichtmodul
6c Lichtmodul
6d Lichtmodul
6e Lichtmodul
6f Lichtmodul
6g Lichtmodul
6h Lichtmodul
6i Lichtmodul
6j Lichtmodul
6k Lichtmodul
7 Lichtstrahl
8a Leuchtfeld
8b Leuchtfeld
8c Leuchtfeld
8d Leuchteld
9 Gehäuse
10a LED
10b LED
10c LED
11a Linse
11b Linse
11c Linse
12a Lichtstrahl
12b Lichtstrahl
12c Lichtstrahl
13a Stromleitung
13b Stromleitung
13c Stromleitung
14 Stromleitung
15 Steuereinrichtung
16 Schwenkachse
17 Schwenkrichtung
18 Schwenkachse
19 Schwenkrichtung
20 Modul
21 Kamera
22 Leerstelle

## Patentansprüche

1. Operationsleuchte (1), umfassend einzelne Lichtmodule (6a bis 6k) mit jeweils einem Gehäuse (9) und in dem Gehäuse (9) untergebrachten Leuchtmitteln (10a bis 10c), wobei die Gehäuse an ihren Seitenflächen oder der Rückseite sowohl mechanische als auch elektronische wie elektrische Verbindungselemente oder Konnektoren aufweisen, um Leuchtmittel in den Lichtmodulen (6a bis 6k) anzusteuern und an eine elektrische Stromversorgung anzuschließen, und um die Lichtmodule, die als Sechsecke ausgebildet sind, zu einer Facettenstruktur zusammenzusetzen, so dass das Lichtfeld die Kontur der Lichtmodulkombination widerspiegelt, wobei die Form der Lichtmodule (6a bis 6g) so gestaltet ist, dass sie auf einer Kugelfläche ohne Zwischenräume augeordnet werden können, und
wobei die Lichtmodule (6a bis 6g) durch beliebige Kombination mit weiteren solchen Lichtmodule zu unterschiedlichsten Gesamtmodulen als Lichtquelle zusammengesetzt werden können, wodurch was die Lichtfeldgröße, die Beleuchtungsstärke und die Lichtfeldform des Lichtfelds verändert werden kann.

2. Operationsleuchte nach Anspruch 1, **dadurch gekennzeichnet, dass** die Leuchtmittel LEDs (10a bis 10c) sind.

3. Operationsleuchte nach einem der vorhergehenden Ansprüche, **dadurch gekennzeichnet, dass** die Lichtmodule (10a bis 10c) schwenkbar miteinander verbunden sind.

4. Operationsleuchte nach einem der Ansprüche 2 oder 3, **dadurch gekennzeichnet, dass** Linsen (11a bis 11c) zur Aufweitung und/oder Vergleichmäßigung des LED-Lichts vorgesehen sind.

## Claims

1. Operating lamp (1) comprising individual light modules (6a through 6k) with one housing (9) each and illumination means (10a through 10c) disposed in the housing (9), wherein the housings comprise on their side surfaces or the rear side mechanical, electronic and electrical connecting elements or connectors to control illumination means in the light modules (6a through 6k) and to connect them to an electric current supply, and to assemble the light modules formed as hexagons to form a facet structure so that the light field reflects the contour of the combination of the light modules,
wherein the shape of the light modules (6a through 6g) is designed such that they can be arranged on a sphere surface without any clearance, and
wherein the light modules (6a through 6g) can be assembled to most different total modules as light source by arbitrary combination with further suchlike light modules,
whereby the size of the light field, the light intensity and the light field shape of the light field can be varied.

2. Operating lamp according to claim 1, **characterized in that** the illumination means are LEDs (10a through 10c).

3. Operating lamp according to any of the preceding claims, **characterized in that** the light modules (10a through 10c) are pivotably connected to each other.

4. Operating lamp according to claim 2 or 3, **characterized in that** lenses (11a through 11c) are provided to widen and/or homogenize the LED light.

## Revendications

1. Lampe chirurgicale (1), comprenant différents modules d'éclairage individuels (6a à 6k) présentant chacun un boîtier (9) et des sources lumineuses (10a à 10c) logées dans le boîtier (9),
les boîtiers présentant sur leurs surfaces latérales ou sur la face arrière des éléments de connexion aussi bien mécaniques qu'électroniques et électriques ou des connecteurs pour commander des sources lumineuses dans les modules d'éclairage (6a à 6k) et les raccorder à une alimentation électrique, et pour assembler les modules d'éclairage, réalisés sous forme d'hexagones, en une structure à facettes, de sorte que la forme du champ lumineux reflète le contour de la combinaison de modules d'éclairage,
sachant que la forme des modules d'éclairage (6a à 6g) est réalisée de sorte qu'ils peuvent être disposés sans interstice sur une surface sphérique,
sachant que les modules d'éclairage (6a à 6g) peuvent être assemblés par combinaison quelconque avec d'autres modules d'éclairage en des modules groupes variés formant sources de lumière, permettant ainsi de faire varier la taille du champ lumineux, l'intensité d'éclairage et la forme du champ lumineux.

2. Lampe chirurgicale selon la revendication 1, **caractérisée en ce que** les sources lumineuses sont des LED (10a à 10c).

3. Lampe chirurgicale selon la revendication 1, **caractérisée en ce que** les modules d'éclairage (6a à 6k) sont reliés entre eux de manière orientable ou pivotante.

4. Lampe chirurgicale selon l'une des revendications 2 ou 3, **caractérisée en ce qu'**il est prévu des lentilles (11a à 11c) pour élargir et/ou homogénéiser la lumière des LED.
